(19) Europäisches Patentamt
European Patent Office
Office européen des brevets

(11) Publication number : 0 136 377 B2

(12) NEW EUROPEAN PATENT SPECIFICATION

(45) Date of publication of the new patent specification : 22.01.92 Bulletin 92/04

(51) Int. Cl.[5] : C07C 2/20, C07C 2/26, C10G 71/04, C10M 107/02

(21) Application number : 83305604.7

(22) Date of filing : 21.09.83

# (54) Oligomerization of olefins and synthetic lubricant comprising olefin oligomers.

(43) Date of publication of application : 10.04.85 Bulletin 85/15

(45) Publication of the grant of the patent : 13.04.88 Bulletin 88/15

(45) Mention of the opposition decision : 22.01.92 Bulletin 92/04

(84) Designated Contracting States : BE DE FR GB IT LU NL SE

(56) References cited :
EP-A- 0 068 554
EP-A- 0 078 346
US-A- 4 239 930
Kirk Othmer Encyclopedia of Chemical Technology, 3rd Edition, p. 748 - 749

(73) Proprietor : TEXACO DEVELOPMENT CORPORATION
2000 Westchester Avenue
White Plains, New York 10650 (US)

(72) Inventor : Hammond, Kenneth George
49 Scenic Hills Drive
Poughkeepsie New York 12603 (US)
Inventor : Sendra, Joseph Charles
c/o Texaco Brasil S.A. Caixa Postal 520
20 001 Rio de Janeiro R.J. (BR)
Inventor : Watts, Lewis William Jr.
9202 Indian Quail Circle
Austin Texas 78758 (US)
Inventor : Marquis, Edward Thomas
9004 Collinfield Drive
Austin Texas 78758 (US)
Inventor : Larkin, John Michael
12414 Dorsett Road
Austin Texas 78759 (US)

(74) Representative : Wood, Anthony Charles et al
Urquhart-Dykes & Lord 91 Wimpole Street
London W1M 8AH (GB)

EP 0 136 377 B2

## Description

The invention relates to processes for the production of oligomers from olefins, and more particularly relates to the production of oligomers from internal olefins by means of a boron trifluoride catalyst. The invention also relates to the use of the oligomerized product as a synthetic lubricant.

Friedel-Crafts catalysts have long been known to oligomerize olefins. For example, see US-A 3 410 925 in which olefins are mixed with alkylatable aromatic hydrocarbons over a Friedel-Crafts catalyst to form an alkylation sludge, which is then mixed with olefins having 3 to 18 carbon atoms which are also passed over the catalyst to produce olefin dimers. US-A-3 652 706 describes the polymerization of olefins having 2 to 20 carbon atoms over a Friedel-Crafts metal halide catalyst plus hydrogen mordenite to produce compounds having a molecular weight between 700 to 2,500. Production of a gasoline fuel composition is described in USA-A 3 749 560, involving reacting a mixture of mono olefins (greater than 50 weight% of alpha olefins) over a Friedel-Crafts catalyst heated to a temperature around 145°C to produce oligomers having molecular weights of 350 to 1,500. Also, US-A 3149178 reveals an improved method for making polymerized olefin synthetic lubricants via a particular distillation technique of oligomers made from alpha mono olefins using a Friedel-Crafts catalyst. Alpha olefins having 6 to 12 carbon atoms may be dimerized in the presence of a Friedel-Crafts catalyst according to the method described in US-A 4172 855.

It is also known that the term "Friedel-Crafts catalysts" includes boron trifluoride, among other metal halide-type Lewis catalysts, see *Kirk-Othmer Encyclopedia of Chemical Technology*, Third Edition, Vol. 11, pg 292. Boron trifluoride has also been known to polymerize olefins, see F. Albert Cotton, et al., *Advanced Inorganic Chemistry: A Comprehensive Text*, Interscience Publishers, 1962, p. 191.

A number of US patents disclose the use of $BF_3$ to oligomerize olefins. Close study will reveal that alpha olefins are considered the only useful form. For example, US-A- 2 780 664 describes the reaction of conjugated dienes with mono alpha and internal olefins over $BF_3$ promoted by an ether mixed with a haloalkane diluent at a temperature from -30 to 100°C to produce oligomers suitable for drying oils. Alpha olefins having from 5 to 20 carbon atoms are oligomerized using $BF_3$ plus an alcohol or water promoter as described in US-A 3 382 291. In this patent, $BF_3$ and a mixture of $BF_3$ plus the promoter complex are introduced in two separate streams. US-A-3 742 082 concerns the dimerization of alpha olefins via $BF_3$, which is promoted with phosphoric acid or water at a temperature from 100 to 150°C. US-A 3 763 244 describes the oligomerization of n-alpha olefins having 6 to 16 carbon atoms over $BF_3$ promoted with water, at a temperature between 10 and 60°C, where it is preferred that $BF_3$ is added continuously.

US-A-3 769 363 describes the oligomerization of olefins having 6 to 12 carbon atoms using $BF_3$ with a carboxylic acid promoter having at least 3 carbon atoms at a temperature between 0 and 20°C, oligomerization being selective to the trimer form. US-A 3 780128 relates to the oligomerization of alpha olefins having 6 to 16 carbon atoms in which $BF_3$ is employed in a molar excess of alcohol. US-A- 3 876 720 describes a two-step procedure by which alpha olefins having 8 to 12 carbon atoms are converted into vinylidene olefins, which are then reacted over a 1:1 molar complex of $BF_3$ and alcohol to produce oligomerized vinylidene olefins. A method for oligomerizing both short and long chain alpha olefins having from 14 to 20 carbon atoms simultaneously over $BF_3$ with an alcohol or water promoter at 0 to 60°C with a monomer recycle is described in US-A-4 225 739. US-A 4 263 465 describes a two-step process for recting 1-butene with a higher alpha olefin over $BF_3$ in the presence of a proton donor at a temperature from -30 to 50°C to produce an oligomer having 8 to 18 carbon atoms. The intermediate oligomer is reacted with other higher alpha mono olefins over the same catalyst system from -30 to 60°C to produce oligomers having 20 to 40 carbon atoms. For more information on $BF_3$-catalyzed oligomerization of alpha olefins, see Brennan, "Wide-Temperature Range Synthetic Hydrocarbon Fluids," Ind. Eng. Chem. Prod. Res. Dev. 1980, Vol. 19, pp 2-6 and Shubkin et al., "Olefin Oligomer Synthetic Lubricants: Structure and Mechanism of Formation" Ind. Eng. Chem. Prod. Res. Dev. 1980, Vol. 19, pp 15-19.

Two patents have been located which involve the reaction of internal olefins over Friedel-Crafts catalysts. US-A 4167 534 describes olefins which are both alpha and internal having from 10 to 15 carbon atoms which are reacted over Friedel-Crafts catalysts between 20 and 200°C to produce oligomers. The only catalysts used in the examples of this patent are $AlCl_3$ and $NaAlCl_4$. The internal olefins are also those that are statistically distributed. Also, the oligomers found useful therein seem to be the hydrogenated bottoms product after the unreacted olefins are removed, without further distillation. US-A4 218 330 describes hydrogenated dimers from alpha olefins having from 12 to 18 carbon atoms, especially 1-tetradecene, made using a Friedel-Crafts catalyst, which includes therein boron trifluoride with a promoter. This method uses predominantly alpha olefins, although the specification mentions that "fairly large amounts of internal olefins can be tolerated without adversely affecting the physical properties of the oligomer". This last remark reveals the general feeling of those working in the field that internal olefins do not produce oligomers whith good properties for synthetic lubricants. For example, US-A 3 952 071 reveals that olefins may be oligomerized in the presence of a mixture of a polyhydric alcohol derivative and an aluminium halide. This patent specification mentions that the olefin could be internal or alpha, but alpha olefins are the only ones used in the examples. US-A- 3 947 509 also claims that internal olefins may be used over a ketone-, ester-, ether- or alcohol-promoted aluminum chloride catalysts, although only alpha olefins are used in the examples.

US-A- 4 300 006 describes a process for producing a hydrocarbon oil by contacting a mixture of alpha and at least 50 weight percent internal olefins with a boron trifluoride dimerization catalyst, but the productivity of useful products is quite low. For example, an alkane diluent is found to be necessary in the process described therein which, in additon to distilling out the lights and the heavies to obtain the lube oil, results in little useful product. Further, this method requires a much longer reaction time and a higher catalyst concentration than desired. It would be beneficial if a method for producing synthetic lubricant componants could be devised which would overcome the aforementioned disadvantages.

In the field of oligomerizing olefins for synthetic lubricants, it is a continual problem to produce olefins having low viscosities at room temperature and below but which have a high viscosity index and low volatility.

EP-A 0078346 provides a process for the oligomerization of internal olefins by disproportionation or metathesis reactions giving rise to non-random distribution of double bonds.

EP-A-0068554 provides a process for oligomerization of mono olefins by contacting a mixture of mono olefins with a catalyst, wherein said mono olefins may comprise a mixture containing at least 99 weight per cent of internal mono olefins having 9 to 24 carbon atoms and having their double bond randomly distributed in their carbon chain, and the catalyst comprises boron trifluoride on a solid substrate (silica, alumina or active carbon) impregnated with phosphoric acid. EP-A-0068554 refers to an Italian Application 20106/80, which was laid open to public inspection on 22nd August 1981 and subsequently granted as IT-A-1141371. This Italian Application relates to the oligomerization of non-terminal olefins with various catalysts, including boron trifluoride/alcohol complexes, to produce bases for synthetic lubricants. Example 7 describes the oligomerization of a $C_{15}$-$C_{18}$ fraction using a $BF_3$/methanol complex. Example 8 describes the oligomerization of a $C_{11}$-$C_{14}$ fraction using a $BF_3$/n- butanol complex.

The present applicants have determined that such a process can be carried out to produce an especially advantageous product, when a mixture of mono olefins having 13 or 14 carbon atoms is oligomerized by boron trifluoride, promoted by 1-butanol, in an autoclave, at a weight ratio of boron trifluoride to 1-butanol of 1.57:1 to 4.1. Contact is preferably effected at 25 to 150°C.

This invention also provides a lubricant composition comprising from 10 to 40 weight% of oligomerized olefins as defined above and minor amounts of conventional lubricant additives. additives.

Preferably the lubricant composition contains 10 to 40 weight percent of the oligomerized olefins, which generally have a viscosity at 99°C of 3.5 to 5.0 centistokes, a viscosity at 25°C of 25 to 40 centistokes, a viscosity index of at least 100 and a thermogravimetric analysis value of greater than 80 weight %.

A suitable formulation for a lubricant composition according to the invention may comprise:

i) from 0.1 to 15.0 wt.% of viscosity index improver,

ii) from 0.01 to 10 wt.% on an oil free basis of a detergent/dispersant system,

iii) from 0.01 to 3.0 wt.% of oxidation and wear inhibitors,

iv) from 10 to 1,000 parts per million of antifoamant, and

v) optionally one or more friction modifiers, rust inhibitors or pour-point depressants.

It has surprisingly been discovered that oligomers which have an unusual combination of properties may be made by reacting predominantly internal mono olefins having 13 on 14 carbon atoms with boron trifluoride and a promoter. It must be stressed that this has not been discovered by any of the other researchers in the field. It is also important to note that predominantly internal olefins are employed in the method of this invention.

The olefins have 13 on 14 carbon atoms. The internal olefins used herein have the double bond randomly distributed across the molecule. In this context, the term "randomly distributed" means that the double bond in the internal olefin is not predominantly in any one location. For example, an olefin mixture comprising a majority of alpha olefins would be outside the scope of this definition since the double bond would be located predominantly between the first and second carbon atoms of the molecules. Likewise, since the internal olefins used for oligomerization in the method of US-A 4 300 006 are made by disproportionation of alpha olefins, the double bond is located predominantly at or near the centre of the molecule, and such olefin feedstocks also fall outside the definition of having a "random distribution" of the double bond. A random distribution includes the distribution one may obtain upon the dehydrogenation of paraffins. One would expect a small amount of alpha olefin to result from such a distribution. However, one would also anticipate that the alpha olefin proportion would be only about 0.1 weight percent, with a maximum value being 1.0 weight percent. As a practical matter, the feedstocks herein can be considered to be entirely internal olefins.

The internal olefins may be generally expressed by the formula RCH=CHR′ where R and R′ are the same or different alkyl radicals of 1 to 11 carbon atoms, but the total number of carbon atoms should not exceed 14

and should not be less than 13. The internal olefin mixtures are potentially more available than the pure cut alpha olefins and are potentially as cheap or cheaper than the corresponding pure cut alphas. It will be shown that the method of this invention affords higher quality products and higher conversions than those obtained with $AlCl_3$ and $AlCl_4Na$ catalysts.

By careful selection of the molecular weight of the feed olefins and the reaction conditions, it was found that a synthetic lubricant base oil with a specific viscosity can be made by the method of this invention which has superior properties over those made by other methods.

It has been found that a base oil having a viscosity of about 4 centistokes at 99°C with excellent properties can be made from internal olefins having 13 or 14 carbon atoms. A "4 centistoke fluid" is a designation given to fluids used in lubricating oil compositions which generally have viscosities of about 4 centistokes at 99°C.

The catalyst is boron trifluoride. 1-butanol 15 used as the promoter. The temperture range at which the oligomerization may be performed successfully is from 25 to 150°C, with an especially prefered range from 65 to 105°C. The pressure range of the reaction may run from atmospheric to 70 bars. The oligomerization of the olefins may be conducted batchwise or continuously.

The lubricating oil compositions of this invention comprise a major amount (preferably 10 to 40 weight per cent) of the synthetic lubricant component of this invention (the hydrogenated olefin oligomers described above), or a mixture of the synthetic lubricant component and a conventional base stock, and minor amounts of additive components.

The conventional base stocks mentioned above, also called lubricating, hydrocarbon mineral base oils, can be paraffinic base, naphthene base or mixed paraffin-naphthene base distillate or residual mineral oils, or synthesized base oils. Paraffin base distillate lubricating oil fractions are the preferred mineral oils for the formulation of crankcase lubricants. The lubricating oil base will generally have been subjected to solvent refining, to improve its lubricating properties and viscosity-temperature relationship, as well as to solvent dewaxing, to remove any waxy components and to improve the pour characteristics of the oil. Suitable synthetic lubricating oils for preparing the lubricants of this invention (these are apart from the internal olefin oligomers discussed previously) include the ester base oils prepared from polyhydroxy alcohols such as pentaerythritol and trimethylolpropane and aliphatic mono-carboxylic acids, the ester base oils prepared from diacids such as dimer acid and azelaic acid and monoalkanols, synthetic hydrocarbons such as polyalpha olefins, and polyglycols and thiols, silicone fluids, polyphenyl ethers and thioethers.

Generally, conventional base stocks having a visocosity at 38°C between 50 and 1,000 SUS, preferably between 70 and 500 SUS can be used in the formulation of the lubricants of this invention. A blend of conventional base oils can be employed, if desired.

The lubricating oil compositions of this invention contain additives to enhance the performance characteristics of the synthetic lubricant component or synthetic lubricant component/conventional base stock mixture. The additives may be any of the suitable standard pour depressants, viscosity index improvers, detergent-dispersants, corrosion, oxidation, and wear inhibitors, antifoamants, or friction modifiers. The choice of the particualr additives to be included in the finished oils and the particular amounts thereof will depend on the use and the conditions desired for the finished oil product.

Specific examples of the supplementary additives are as follows:

Viscosity Index Improvers and Pour Depressants

A widely used and suitable viscosity index improver/pour depressant is the polymethacrylate having the general formula:

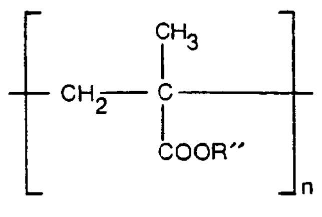

where R" is an aliphatic radical, having from 1 to 20 carbon atoms and n is 600 to 35,000. One of the most suitable viscosity index improvers is the dispersant polymethacrylate tetrapolymer of butyl methacrylate, dodecyl methacrylate, octadecyl methacrylate and dimethylaminoethyl methacrylate having a respective component weight ratio in the polymer of about 4:10:5:1. Another viscosity improver is a copolymer of ethylene and 30 to 50 weight percent of propylene having a molecular weight of 20,000 to 50,000, or the derivative thereof

having pendant organo-nitrogen functionality contributing 0.1 to 0.5 weight percent nitrogen to the polymer. The viscosity index improvers and/or pour depressants are normally employed in the finished lubricant compositions in quantities from 0.01 to 15 percent by weight.

Detergent-Dispersants

Examples of detergent-dispersants which can be employed are the ethoxylated inorganic phosphorus acid-free, steam-hydrolyzed, polyisobutene-$P_2S_5$ dispersants; the adducts formed from neopentylpolyol inorganic phosphorus acid-free, steam-hydrolyzed polyisobutene-$P_2S_5$ reaction product as further described in US-A-3 281395, the $C_{50}$-$C_{200}$ alkenyl succinimide derivatives of alkene polyamines of the type described in US-A-3172 892 and 3 210 383. The succinimide derivatives have the formula:

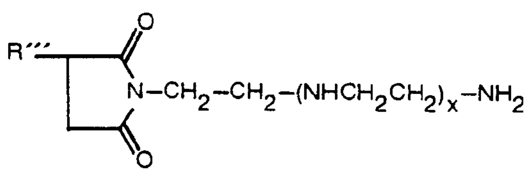

wherein R''' is alkenyl having from 50 to 2,000 carbon atoms, and x is 0 to 10. Particularly suitable examples are where R''' is polyisobutene having a molecular weight of 1,000 to 1,500, and x is 4 or 5, and mixtures thereof. Further detergent-dispersants include the alkaline earth metal alkylphenolates, such as barium nonylphenolate, barium dodecylcresolate, calcium dodecylphenolate and the calcium carbonate overbased calcium alkaryl sulfonates formed by blowing a mixture of calcium hydroxide and calcium alkaryl sulfonate; e.g., calcium alkylbenzene sulfonate of about 900 molecular weight with carbon dioxide to form a product having a total base number (TBN) of 50 or more, typical values being 300 to 400. Detergent-dispersants are present in the finished compositions of the invention in amounts from 0.1 to 15.0 weight percent.

Oxidation, Corrosion and Wear Inhibitors

Commonly employed lube oil oxidation and wear inhibitors are the divalent dialkyl dithiophosphates resulting from the neutralization of a $P_2S_5$-alcohol reaction product with a divalent metal or divalent metal oxide. Barium and zinc dialkyl dithiophosphate are specific examples. Another class of antioxidants comprises the polyalkylated diphenylamines, such as a mixture of 2,2′-diethyl-4,4′-dioctyl-diphenylamine and 2,2′-diethyl-4-octyl-diphenylamine. A further class of antioxidants comprises the polyalkylated and the polyalkylated methylene bridged phenols. Commonly employed corrosion inhibitors include the ethoxylated alkylphenol derivatives. The corrosion, oxidation and wear inhibitors are usually present in the finished lubricating oil compositions in concentrations of 0.01 to 3 weight percent.

Antifoamants

If antifoamants are employed in the finished compositions, one widely used class which is suitable are the dimethyl silicone polymers, typically employed in amounts of 10 to 1,000 ppm.

There are many other additives which may be useful in a lubricating oil composition as encompassed by this invention. Many of these componants are listed in the disclosures of US-A-3 864 270; 4169 799; 4194 981; and 4 253 980, which patents are themselves concerned with novel additives.

The invention will be further illustrated with reference to the following Examples. First, the experimental methods will be described, and then the results will be tabulated.

Comparative Examples

A number of comparative oligomerization examples were run using the procedures of US-A- 4167 534. It is believed that this patent constitutes the closest prior art. The examples herein were patterned after Examples 1, 5 and 7 of US-A-4 167 534. These examples were chosen because they represented a wide variety of conditions, particularly temperature. The primary variable in the comparative examples is the olefin feedstock, although sometimes twice the amount of $AlCl_3$ used in US-A-4 167 534 is employed in an attempt to improve the conversion.

According to the disclosure of US-A-4 167 534, Example 1 is begun by heating the feedstock to 80°C. 1

% $AlCl_3$ is then added over 15 minutes. The temperature is then raised to 100°C and maintained at this level for 100 minutes. The product is then discharged, separated from the heavy catalyst layer, washed with caustic solution and then distilled.

Example 5 begins by adding 1 % $AlCl_3$ in only one portion to the olefin feedstock at room temperature. The temperature is allowed to rise autogenously for 120 minutes. The product is then discharged, separated from the heavy catalyst layer, washed with caustic solution and distilled.

5% $NaAlCl_4$ is then added to the feedstock of Example 7 of US-A 4167 534 at 130°C over 90 minutes. The reaction mass is then maintained at 130°C for a further 60 minutes. The product is then discharged, separated from the heavy catalytic layer, washed with caustic solution and distilled. The results of the comparative examples are shown in Table 1 below.

## TABLE 1

Comparative Examples

| Example | Reaction Feed | Conditions Catalyst | Temperature, °C | Method of US—A 4 167 534 Used | Conversion Basis LC | % Bottoms Basis Olefin Charged | Properties of Hydrogenated Stripped Oligomer: Kinematic Viscosity, centistokes, 99°C | Kinematic Viscosity, centistokes, 25°C | VI | Pour Point, °C | TGA: % Sample Remaining at 233°C |
|---|---|---|---|---|---|---|---|---|---|---|---|
| 1 | $C_{13-14}$ int. | $AlCl_3$ | 80 | Ex. 1 | 32.5 | 21.6 | 5.69 | 56.00 | 127.4 | <-45 | 91.9 |
| 2 | $C_{13-14}$ int. | $AlCl_3$* | 80 | Ex. 1 | 42.2 | 29.2 | 5.85 | 60.96 | 121.4 | <-45 | 91.3 |
| 3 | $C_{11-14}$ int. | $AlCl_3$ | 80 | Ex. 1 | — | 21.0 | 6.79 | 80.39 | 116.9 | <-45 | 88.5 |
| 4 | $C_{14}$ alpha | $AlCl_3$ | 80 | Ex. 1 | 85.9 | 80.2 | 22.07 | 341.83 | 129.9 | <-5 | 98.2 |
| 5 | $C_{13-14}$ int. | $AlCl_3$ | R.T.** | Ex. 5 | 24.8 | 16.8 | 6.40 | 68.90 | 124.4 | <-45 | 92.2 |
| 6 | $C_{13-14}$ int. | $AlCl_3$* | R.T. | Ex. 5 | 45.6 | 27.4 | 6.43 | 71.75 | 122.1 | <-45 | 91.4 |
| 7 | $C_{11-14}$ int. | $AlCl_3$ | R.T. | Ex. 5 | 36.8 | 23.2 | 7.12 | 86.72 | 116.5 | <-45 | 89.5 |
| 8 | $C_{14}$ alpha | $AlCl_3$ | R.T. | Ex. 5 | 93.9 | 87.6 | 18.20 | 260.91 | 131.2 | <-10 | 98.3 |
| 9 | $C_{13-14}$ int. | $NaAlCl_4$ | 130 | Ex. 7 | 42.0 | 30.3 | 5.20 | 48.68 | 125.9 | <-45 | 88.2 |
| 10 | $C_{11-14}$ int. | $NaAlCl_4$ | 130 | Ex. 7 | 68.9 | 44.8 | 6.96 | 87.04 | 114.0 | <-40 | 91.3 |
| 11 | $C_{14}$ alpha | $NaAlCl_4$ | 130 | Ex. 7 | 100.0 | 87.1 | 19.44 | 286.80 | 130.5 | >-10 | 96.0 |

*Twice as much $AlCl_3$ was used in these examples as described in the examples of US—A—4 167 534
**R.T. means room temperature

In the results outlined in Table 1, the conversion is weight percent of monomer oligomerized, as determined by liquid chromatography. The weight percent of the bottoms as based on the olefin charged is given in the next column. While these two columns of data generally measure the same concept (notice the qualitative correlation), the applicants prefer to use "% conversion" while US-A-4 167 534 uses the "% bottoms" method. Both are employed in the examples of Table 1 for comparative purposes.

Kinematic viscosities at two standard temperatures are given in centistokes. The viscosity index (VI) is the change in viscosity with temperature such that the higher the number, the lower is the change in viscosity with temperature. Conversely, a low VI signifies a large change in viscosity with temperature. Pour point is a measure of the lowest temperature, in degrees Celsius, at which the sample will begin to pour. Below that temperature, the composition may generally be regarded as a solid. The thermogravimetric analysis (TGA) is a measure of volatility via measuring the weight percent of sample remaining at 233°C as the temperature is raised in a slow, uniform manner, usually 10°C per minute. An oligomeric product should have a TGA analysis with at least 80% of the sample remaining at 233°C in order to have sufficiently low volatility to be useful as a base stock for lube oil formulation.

Examples illustrating the Invention

The following examples illustrate the method of the invention using $BF_3$ as the catalyst and only internal olefins as the olefin feedstock. Two examples are included using $C_{14}$ alpha olefin as the feedstock to show that an unsuitable oligomer mixture is produced. The oligomer from alpha olefins having 14 carbon atoms (1-tetradecene) was deficient in its low temperature properties for use in crankcase engine oils. Generally, the viscosity at 25°C should be 30 to 30.5 centistokes (cs) or less while the $C_{14}$ alpha olefin oligomer was observed to have a viscosity in the 32-33 cs range. The pour point of a synthetic lubricant base oil candidate should be less than -45°C. These differences are exaggerated at -30°C (the temperature at which cold cranking viscosity tests are done) and would result in too viscous a fluid at cold temperature.

Preferably, a "4 centistoke fluid" (measured at 99°C) should have a viscosity of 25 to 40 centistokes at 25°C, a viscosity of 3.5 to 5.0 centistokes at 99°C, a viscosity index of greater than 100, a pour point of less than -45°C and a thermogravimetric analysis percent remaining at 233°C value of greater than 80 weight percent. It is especially preferred that 4 centistoke fluids have a viscosity of between 25 and 34 centistokes at 25°C, a viscosity between 3.5 and 4.5 cs at 99°C, a minimum viscosity index of 110, a maximum pour point of less than -45°C and a thermogravimetric analysis (TGA) value of 86%, minimum.

Example 12

A solution of 43.8 g of $C_{13-14-15}$ random internal linear olefins (53.4% $C_{13}$, 45.0% $C_{14}$, 0.5% $C_{15}$, 1.1 % paraffins) and 0.12 g of 1-butanol in a nitrogen atmosphere was saturated with $BF_3$ at 25°C by slow sparging for 25 minutes. Pot contents were maintained at 25-27°C by external cooling, while 55.6 g of a solution of 55.0 g of $C_{13-14-15}$ olefins and 0.8 g of 1-butanol was added over 100 minutes. Boron trifluoride saturation was maintained during this addition and for 105 minutes thereafter. $BF_3$ introduction was stopped and the pot contents were heated to 86°C over a 105 minute period, and maintained at 85-86°C for 30 minutes. After cooling, 110 ml of $H_2O$ was added; the contents were stirred rapidly for 20 minutes, and the top layer was removed. It was washed with 115 ml of $H_2O$ and stripped on a rotary evaporator at 4000 Pa to a maximum bath temperature of 95°C; 96.9 g of a clear light yellow liquid remaining (98.3% recovery). Analysis by gel permeation chromatography indicated 61.34% dimer, 25.13% trimer, and 12.95% monomer, the balance being higher oligomers.

Example 13

The product of Example 12 (93.8 g) was hydrogenated over 9.60 g of a powdered Ni/Cu/Cr catalyst, which is described in US-A 3152 998. The conditions included a pressure of 125 bars and a temperature of predominantly 208°C (with temperature briefly reaching 316°C).

Vacuum stripping was conducted at 120 PA with a maximum head temperature of 105°C and a maximum pot temperature of 157°C, to remove 11.89 g of lighter components (>91 % monomers). The hydrogenated bottoms product consisted (GPC analysis) of 26.7% trimer, >67.8% dimer, and >3.41 % monomer. The kinematic viscosities at 25°C, 38°C, and 99°C were 29.57, 18.26 and 3.89 centistokes, respectively. The pour point was measured to be less than -45°C. Thermogravimetric analysis indicated 81.3% of the sample remained at 233°C.

Example 14

This is essentially the same procedure as Example 12, except that $Bf_3$ introduction was stopped when olefin/1-butanol introduction stopped. The heating period was for 2 hours at 90°C. GPC analysis indicated 19.8% trimer, 62.2% dimer and 17.94% monomer, the balance being higher oligomers.

Hydrogenation

In order to form materials which have adequate oxidative stability for lubricants, the oligomerized olefins are optionally hydrogenated, either partially or totally. This hydrogenation is done by procedures known to those skilled in the art as exemplified by US-A 4 045 508; 4 013 736; 3 997 622 and 3 997 621. A particularly preferred catalyst for this hydrogenation is a nickel-copperchromia catalyst described in US-A 3152 998. As is well known, such hydrogenations may be performed either batchwise or continuously.

When the method of the present invention was scaled up for a pilot plant run, it was discovered that the resulting oligomer mixture did not give the expected desirable properties seen in the lab scale experiments. In the pilot plant scale-up, stripping out the monomer was performed first and then hydrogenation was conducted over the nickel-copper-chromia catalyst described above. Monomer removal was performed before hydroganation and monomers were recycled to the oligomerization step. During the pilot plant stripping step, however, over 50% of the oligomeric material came off overhead at temperatures from 210 to 282°C in an attempt to obtain a material with a good TGA (volatility) value. Apparently, some of the unhydrogenated oligomer mixture was thermally unstable, and portions of it were reverting to monomers or intermediates and distilling off as volatiles. It is, therefore, important that monomer removal be accomplished under conditions as mild as possible; that is, the reboiler or pot temperature should preferably be kept at or under 180°C when stripping out monomer.

While the methods of others in the field include a distillation step after hydrogenation, to obtain products having various viscosities at 99°C, it is much preferred in the method of the invention that no further distillation be conducted. In other words, the monomer-stripped, hydrogenated bottoms are the desired synthetic lubricant components. Thus the method of this invention does not require the customary distillation step, yet surprisingly produces a synthetic lubricant component that has excellent properties and performs in a superior fashion. It is also anticipated however, that one skilled in the art may find subsequent distillation useful in the practice of the method of this invention.

Examples 15-26

The following examples illustrating the method of the invention were conducted according to one of the following procedures Examples 15, 19 and 24 illustrate the specific combination of reaction parameters employed according to the invention, the other Examples being comparatives.

Procedure A

Examples 15 to 18 used the following experimental procedure. To a 300 ml stainless steel autoclave was charged 158.6 g of olefin and 1.4 g of 1-butanol. The autoclave was sealed and heated to approximately 98°C at which time $BF_3$ gas was introduced in amounts ranging from 3.1 to 4.4 g (average 3.8 g $BF_3$ per run). The reaction mixture was stirred and allowed to generate heat on its own (no cooling). The reaction mixture was stirred for 60 minutes (time measured from first $BF_3$ addition, and $BF_3$ was added over a 3-6 minute period). Cooling water was then turned on. The cooled reaction mixture was neutralized with 10 grams of $Na_2CO_3$ and 100 ml of $H_2O$. After layer separation, the organic layer was washed twice more with fresh water and dried. The oligomer was analyzed (GPC/LC) for conversion and subjected to hydrogenation at 210°C (2 hours), 140 Bars $H_2$ pressure in the presence of a nickel-copper-chromium oxide catalyst (5% by weight basis weight oligomer). Stripping the oligomers of monomer was performed after hydrogenation in all of Examples 15-26 in a manner similar to that of Example 27.

Procedure B

Examples 19 to 23 used procedure B, which was identical to procedure A except that 0.7 g to butanol was used (instead of 1.4 g) and the amount of $BF_3$ added ranged from 2.2 to 2.8 g with a 2.5 g average.

Procedure C

Examples 24 to 26 used procedure C, which was identical to procedure A except that the temperature of the reaction mixture (olefin and promoter) before $BF_3$ addition was 65°C (instead of 98°C) and the amount of $BF_3$ added ranged from 2.2 g to 4.0 g (3.0 g average). Conversions and properties of the oligomers are summarized in Table 2 below.

It should be noted that the amount of catalyust used in the method of this invention (1.4 to 2.8 weight percent of the olefin feed) is notably less than the amount of catalyst used in other methods in the field, such as the method disclosed in US-A 4 300 006 (2.6 to 6.1 weight percent). In further contrast with the method of this particular patent, no employment of a diluent, no heavies or lights (except monomers) were removed, and a shorter reaction time are features of the inventive method. Another difference lies in the fact that the method of US-A 4 300 006 uses a mixture of alpha and internal olefins having carbon numbers that are quite different from each other, unlike the instant method.

TABLE 2

Examples illustating the Invention

Properties of Hydrogenated Stripped Oligomer

| Example | Reaction Conditions Feed | Reaction Conditions Catalyst | Temp. °C | Procedure | Con-version Basis LC | Kinematic Viscosity, centistokes, 99°C | Kinematic Viscosity, centistokes, 25°C | VI | Pour Point, °C | TGA: % Sample Remaining at 233°C |
|---|---|---|---|---|---|---|---|---|---|---|
| 15 | $C_{13-14}$ int. | $BF_3$-butanol | 98 | A | 90.5 | 3.82 | 29.67 | 114.4 | <-45 | 89.5 |
| 16 | $C_{11-14}$ int. | $BF_3$-butanol | 98 | A) | | | | | | |
| 17 | $C_{11-14}$ int. | $BF_3$-butanol | 98 | A) Avg. | 83.7 | 4.45 | 40.79 | 106.8 | <-45 | 89.2 |
| 18 | $C_{11-14}$ int. | $BF_3$-butanol | 98 | A) | | | | | | |
| 19 | $C_{13-14}$ int. | $BF_3$-butanol | 98 | B | 86.4 | 3.68 | 29.17 | 118.1 | >-35 | 88.8 |
| 20 | $C_{11-14}$ int. | $BF_3$-butanol | 98 | B) | | | | | | |
| 21 | $C_{11-14}$ int. | $BF_3$-butanol | 98 | B) avg. | 53.4 | 4.04 | 34.48 | 105.0 | <-45 | 84.9 |
| 22 | $C_{11-14}$ int. | $BF_3$-butanol | 98 | B) | | | | | | |
| 23 | $C_{14}$ alpha | $BF_3$-butanol | 98 | B | 89.1 | 4.27 | 33.09 | 134.3 | <-35 | 98.8 |
| 24 | $C_{13-14}$ int. | $BF_3$-butanol | 65 | C | 83.4 | 3.86 | 29.90 | 119.4 | <-45 | 90.4 |
| 25 | $C_{11-14}$ int. | $BF_3$-butanol | 65 | C | 77.5 | 5.19 | 52.54 | 115.6 | <-45 | 93.8 |
| 26 | $C_{14}$ alpha | $BF_3$-butanol | 65 | C | 83.6 | 4.41 | 32.70 | 148.6 | >-35 | 93.5 |

It may be noted from inspection of Tables 1 and 2, that alpha olefins produce invariably poorer oligomer mixtures than do internal olefins. No examples have higher viscosities at both 99°C and 25°C than do the alpha olefin oligomers from Examples 4, 8 and 11. It must be remembered that the method of the present invention has the requirement of using only internal olefins to obtain low viscosities, a feature not found in any related method. It should also be pointed out that oligomers made from alpha olefins (Examples 4, 8, 11, 23 and 26) have rather high pour points, which make them unacceptable for use in synthetic lubricants. The higher pour point of Example 19 is thought to be an erroneous data point.

Secondly, it may be noted that the viscosities of the $BF_3$ catalyzed oligomers are all lower than those produced by the method of US-A 4167 534. In addition, the conversions are much higher in the $BF_3$ runs. It is particularly surprising that $C_{13-14}$ internal olefins (Examples 15, 19 and 24), having a higher average molecular weight than the $C_{11-14}$ internal olefins (Examples 16-18, 20-22 and 25) produce oligomer mixtures having a *lower* viscosity than the mixtures from $C_{11-14}$ internal olefins. These examples show how the choice of molecular weight range of the olefin feedstock greatly affects the properties of the product oligomers.

It is surprising that such low viscosities (relative to other methods) may be found in oligomer mixtures that also have low pour points and viscosity indexes and volatilities comparable with those of other methods. It is precisely such a combination of advantageous properties that is being sought after in the field and which has not been discovered until now.

In addition, it should be noted that the method revealed in US-A 4 300 006 requires that the dimerization feedstock be obtained from the disproportionation of alpha olefins having 8 to 10 carbon atoms. As a result, the dimerization feedstocks used therein are a mixture of alpha and internal olefins where the alpha olefins have slightly more than half the carbon number of the corresponding internal olefins and the internal olefins are highly symmetrical (being formed from the disproportionation of two alpha olefins). For example, runs IX and XII therein oligomerize $C_{14}$, $C_{16}$ and $C_{18}$ internal olefins where the double bond is at or near the centre of the olefin molecule. The method of the present invention uses internal olefins where the double bond is randomly distributed, instead of located near the centre of a symmetrical mono olefin. These differences in the feedstocks cause important differences in the properties of the resulting oligomers, as shown by the following Examples involving $C_{14}$ internal olefin and $C_8$ alpha olefin as a mixed feedstock.

Example 27

Oligomerization

The oligomerization of a 70 weight percent $C_{14}$ internal olefin and 30 weight percent $C_8$ alpha olefin mixture was accomplished over 2.5 g of a boron trifluoride catalyst with 1.1 g of 1-butanol as a protonic promoter and initiated at 95.1°C.

To a dry and clean 300 ml Hastelloy C autoclave were added 119 g of $C_{14}$ internal olefin from Shell Chemical Company's Higher Olefin Process (SHOP). The double bond in these internal olefins is randomly distributed throughout the molecule. Added at the same time were 51 g of $C_8$ alpha (1-octene from Aldrich Chemical Company, Inc.). At the time, this was the closest available approximation of the feedstock of US-A 4 300 006. These additions were followed by 1.1 g of 1-butanol promoter. The autoclave was sealed and the contents were heated to 95.1°C with stirring. Starting at 95.1°C, $BF_3$ gas was introduced by adding four shots of $BF_3$ over an 11 minute period (2.5 g total $BF_3$ added) to the stirred reaction mixture. At the end of 17 minutes (measured from the first $BF_3$ addition), the temperature had risen 110.2°C for a maximum exotherm of 15.1°C. One hour after the first $BF_3$ addition the reaction temperature was 101.5°C. The heat was turned off and cooling water was turned on. The reaction mixture was neutralized with an aqueous $Na_2CO_3$ solution and washed twice more with water. The organic layer was separated and dried by filtering through folded filter paper to obtain a net weight of 156.3 g. Liquid chromatography analysis indicated 31.9% of the material was $C_8$, $C_{14}$ or $C_{16}$, and 27.5% was dimer $C_{22}$ (from $C_8$ and $C_{14}$), and 32.2% was dimer $C_{28}$ (from $C_{14}$ and $C_{14}$), while 8.4% was $C_{36}$ or heavier. Conversion to material lighter than $C_{36}$ was about 68.1%. The weight ratio of dimer to trimer and heavies was 7.19:1.

Hydrogenation and Stripping

A 1 litre stirred stainless steel autoclave was charged with 144.5 g of oligomer from the previous step and 7.2 g of a nickel-copper-chromium oxide hydrogenation catalyst. The autoclave was flushed with hydrogen three or four times, and pressurized to 70 Bars with hydrogen. Subsequently, the autoclave was heated to 210°C (the pressure increased to only 83.7 Bars) and pressurized again to 139 Bars with hydrogen. The reaction mixture was stirred at 210°C for four hours during which the pressure remained at 139 Bars. The hydrogenated oligomer was filtered and 137.3 g of it was subjected to high vacuum stripping. The material was distilled through a jacketed column (with about 40 cm of Goodloe packing) until the head temperature reached 105°C at 8 Pa. The bottoms weighed 67.1 g (49.6% of the total material, overhead plus bottoms) and the overhead weighed 68.3 g (50.4% of the total material). The bottoms product had a viscosity of 3.6 cs at 99°C, a 25°C viscosity of 27.4 cs, a pour point of <-45°C and a viscosity index of 110. Liquid chromatography analysis indicated the presence of 25.5% of dimer ($C_{22}$), 60.2% of dimer ($C_{28}$) and 14.3% of heavier materials. The TGA of the bottoms product indicated that volatility was moderately high (85.0% of the sample remained at 233°C in TGA of 10°C/minute).

Example 28

Oligomerization

Oligomerization of a 70% $C_{14}$ internal olefin - 30% $C_8$ alpha olefin mixture catalyzed by 2.2 g of $BF_3$ with 1.1 g of 1-butanol as a promoter was initiated at 94.9°C. As in the previous example, 119 g of $C_{14}$ internal olefin were added to a 300 ml autoclave along with 51 g of $C_8$ alpha olefin followed by 1.1 g of 1-butanol. The autoclave was sealed and heated to 94.9°C. Starting at 94.9°C, $BF_3$ gas was added over an 11 minute period (totalling 2.2 g of $BF_3$) to produce a 15.1°C maximum exotherm after 16 minutes had elapsed after the first $BF_3$ addition. After a one hour reaction time, measured from the first $BF_3$ addition, the mixture was cooled and neutralized with aqueous sodium carbonate. The organic layer was separated and dried by filtering through folded filter paper, to give a net weight of 162.5 g. Liquid chromatography analysis indicated 31.1% of the material was $C_8$, $C_{14}$ or $C_{16}$, 27.2% was dimer $C_{22}$ and 33.4% was dimer $C_{28}$ while 8.3% was $C_{36}$ or heavier. Conversion to materials higher than $C_{16}$ was 68.9%. The weight ratio of dimer to trimer and heavies was 7.30:1.

Hydrogenation and Stripping

From the above step, 145.0 g of the oligomer was hydrogenated over 7.2 g of nickel-copper-chromium oxide catalyst. The hydrogenation was conducted at 210°C and 139 Bars for four hours. It was followed by filtration and stripping as described in the previous example. The bottoms products amounting to 55.3% of the charge had a viscosity of 25.7 cs at 25°C and a viscosity of 3.45 cs at 99°C. The pour point of the bottoms material was unacceptably high, -40°C, and the viscosity index was 109.0. Liquid chromatography analysis indicated 33.6% of dimer $C_{22}$, 53.8% of dimer $C_{28}$ and 12.6% heavies. The weight ratio of dimer to trimer and heavies was thus 6.94:1. The TGA indicated 82.1 % sample remaining.

Example 29

Oligomerization

Oligomerization of a 70% $C_{14}$ internal olefin - 30% $C_8$ alpha olefin mixture catalyzed by 2.5 g $BF_3$ and 1.1 g 1-butanol as promoter was conducted starting at 75.1°C. To a clean and dry 300 ml autoclave were added 119 g of $C_{14}$ internal olefin and 51 g of $C_8$ alpha olefin, from the same sources as the previous two examples, followed by 1.1 g of 1-butanol promoter. The autoclave was sealed and heated to 75.1°C, and at that temperature $BF_3$ gas was added in increments (shots) over a 10 minute period. Five separate shots were applied to total 2.5 g. Eleven minutes after the first $BF_3$ addition, the reaction temperature had risen to 100.7°C (a maximum exotherm of 25.6°C). The reaction was held at 75°C for 1.5 hours total, and then cooled and worked up as in the previous examples. The dry product from this lower temperature oligomerization had the following liquid chromatography analysis: 12.7% of monomer ($C_8$, $C_{14}$ and $C_{16}$), 23.7% of $C_{22}$, 42.1 % of $C_{28}$ and 21.5% of trimer and heavies. Conversion to materials greater than $C_{16}$ was 87.3% with the weight ratio of dimer to trimer and heavies being 3.07: 1.

Hydrogenation and Stripping

Hydrogenation of the oligomer from the above step was completed at 210°C for 4.0 hours at 139 Bars hydrogen pressure. Workup (filtration), followed by high vacuum stripping, afforded a bottoms product which amounted to 70.7% of the charge and had the following properties: viscosity of 4.16 cs at 99°C, viscosity of 34.5 cs at 25°C, pour point of <-45°C and a viscosity index of 124.2. The liquid chromatography analysis indicated 16.7% of the material was $C_{22}$, 56.8% was $C_{28}$ and 26.5% was heavies. TGA indicated the sample had excellent volatility (90% remaining at 233°C).

Examples 30-38

Examples 30 to 35 were conducted in a manner similar to Examples 27 to 29, except that certain parameters were changed as shown in Table 3.

Examples 36 to 38 were conducted according to the following procedure. Eighty-three grams of $\Delta^7$-$C_{14}$ and 36 g of $\Delta^9$-$C_{18}$ internal olefin and 51 g of $C_8$ alpha olefin were added to a 300 ml autoclave followed by 1.1 g of 1-butanol. This olefin mixture is the closest approximation to the feedstocks of US-A 4 300 006 obtainable with the materials on hand. The autoclave was sealed and $BF_3$ was introduced in the indicated quantities. Workup was conducted as usual, involving an aqueous $Na_2CO_3$ wash followed by two water washes, and filtering the organic layer through filter paper to dry it. Hydrogenation was accomplished at 210°C and in the presence of 5% (by weight, basis olefin) nickel catalyst and 139 Bars hydrogen pressure for four hours. The hydrogenation product was filtered and distilled at high vacuum (<13 Pa) and to a head temperature of 110°C. The bottoms product was submitted for analysis. The results of this last set of comparative examples are summarized in Table 3 below.

TABLE 3

Experiments using lower molecular weight alpha and higher molecular weight internal olefins as feedstocks

| Ex. | Feedstock, wt.%, Internal | Alpha | Reaction Temp., °C | Reaction Time Hours | $BF_3$ Added, Grams | Maximum Exotherm, °C | Liquid Chromatography Conv. | Dimer, Trimer and Heavies | % Bottoms | Properties of Hydrogenated Stripped Oligomers: Kin. Visc., Centistokes 99°C | 25°C | VI | Pour Point, °C | TGA |
|---|---|---|---|---|---|---|---|---|---|---|---|---|---|---|
| 27 | 70 $C_{14}$ | 30 $C_8$ | 95.1-110.2 | 1.0 | 2.5 | 15.1 | 68.1 | 7.19:1 | 49.6 | 3.80 | 27.4 | 110.0 | <−45 | 85.0 |
| 28 | 70 $C_{14}$ | 30 $C_8$ | 94.9-110.0 | 1.0 | 2.2 | 15.1 | 68.9 | 7.30:1 | 55.3 | 3.45 | 25.7 | 109.0 | −40 | 82.1 |
| 29 | 70 $C_{14}$ | 30 $C_8$ | 75.1-100.7 | 1.5 | 2.5 | 25.6 | 87.3 | 3.07:1 | 70.7 | 4.16 | 34.5 | 124.2 | <−45 | 90.0 |
| 30 | 70 $C_{14}$ | 30 $C_8$ | 75.0-96.4 | 1.5 | 2.5 | 21.9 | 93.5 | 1.55:1 | — | — | — | — | — | 86.0 |
| 31 | 48.8Δ7 $C_{14}$ 21.2 Δ9 $C_{18}$ | + 30 $C_8$ | 75.1-97.4 | 1.5 | 2.1 | 22.3 | 83.1 | — | 83.5 | 3.41 | 24.3 | 108.9 | <−5 | 76.4 |
| 32 | 70 $C_{14}$ | 30 $C_8$ | 25.0-47.8 | 1.5 | 2.6 | 22.8 | 82.0 | 3.74:1 | 81.9 | 4.91 | 40.8 | 138.6 | <−25 | 92.9 |
| 33 | 70 $C_{14}$ | 30 $C_8$ | 25.0 28.1 | 6.5 | 2.4 | 3.1* | 21.4 | 6.38:1 | 15.5 | — | — | — | — | 83.8 |
| 34 | 70 $C_{14}$ | 30 $C_8$ | 25.0-54.5 | 1.5 | 5.4 | 29.4 | 96.9 | 0.93:1 | 93.5 | 4.99 | 45.0 | 125.6 | <−35 | 92.0 |
| 35 | 70 $C_{14}$ | 30 $C_8$ | 25.0-55.8 | 6.5 | 5.4 | 30.7 | 97.3 | 0.65:1 | 92.8 | 5.91 | 58.4 | 126.9 | <−40 | 93.6 |
| 36 | 48.8 Δ7 $C_{14}$ 21.1 Δ9 $C_{18}$ | + 30 $C_8$ | 25.2-44.4 | 1.5 | 2.5 | 19.2 | 86.1 | — | 72.0 | 4.40 | 37.1 | 120.3 | <−45 | 90.3 |
| 37 | " | " | 25.1-41.7 | 1.5 | 5.1 | 21.6 | 93.1 | — | 85.0 | 4.67 | 39.2 | 123.6 | <−45 | 90.0 |
| 38 | " | " | 25.1-51.1 | 6.5 | 5.5 | 26.0 | 96.4 | — | 85.2 | 5.63 | 54.0 | 123.2 | <−40 | 94.0 |

* This reaction never started.

As can be seen from Table 3, the product from the feedstocks used in US-A-4 300 006 are unsuitable for use as a synthetic lubricant without further distillation; i.e., the bottoms product found useful suing the internal olefin feedstocks of the invention is superior. Examples 32-38 have 99°C and/or 25°C viscosities and/or pour points which are too high for use as 4 cs synthetic lubricants. One skilled in the art would not expect these materials to pass cold cranking tests. These same feedstocks, when run at a higher temperature, produce a material with a low viscosity index and a poor TGA value (see Example 31).

Thus, it is determined by comparative examples using the conditions of US-A-4 300 006 that the resulting products would need to be distilled in order to meet the 4.0 cs 99°C viscosity requirement and apparently the cold cranking specifications as well.

The oligomer mixtures produced from $C_{13-14}$ internal olefins via a promoted $BF_3$ catalyst have proven to be exceptional synthetic lubricant additives. As these mixtures have a viscosity of about four centistokes at 99°C, they are considered "4 cs" fluids. A typical fluid of this invention was compared with the commercially available 4 centistoke decene-1 derived polyalpha olefin (PAO). It should be emphasized that the synthetic lubricant components of the instant invention are preferably used without further distillation after the monomer removal and hydrogenation steps. In other words, the undistilled bottoms are the finished synthetic lubricant component. The polyalpha olefins must be distilled into "2 cs", "4 cs", "6 cs", etc. fractions before they can be useful. Thus, the method of this invention does not require a costly distillation step, which is an important advantage over methods used by others in the field. Comparison of the properties of the fluids themselves are given in Table 5. It may be seen that the fluid of this invention (A) is somewhat less viscous than the polyalpha olefin fluid (B) at the higher temperatures, though it is somewhat more viscous at the lower temperatures. The viscosity index for fluid A is somewhat less than for fluid B, but they are generally comparable.

Example 39

A SAE 5W-30 grade lubricating oil composition was derived by combining the following components:

| Weight Percent | |
|---|---|
| 35.00 | Four centistoke synthetic lubricant component (described previously) |
| 0.51 | Dispersant olefin copolymer VI improver |
| 0.08 | Polymethacrylate |
| 0.04 | Nitrogen as an alkenyl succinimide |
| 0.24 | Calcium as an overbased calcium sulfonate |
| 0.12 | Zinc as a zinc dialkyldithiophosphate |
| 0.25 | Alkylated diphenylamine |
| 0.25 | Alkylated phenol |
| 0.05 | Ethoxylated alkylphenol |
| 0.50 | Friction modifier |
| 150 ppm | Silicone polymer |
| Balance | Mineral oil (~300 SUS at 100°F) |

This is the formulation A whose properties are described in Tables 4 and 7 below.

Example 40

A SAE 10W-30 grade lubricating oil composition was derived by combining the following components:

| Weight Percent | |
|---|---|
| 33.0 | Four centistoke synthetic lubricant component (described previously) |
| 0.85 | Ethylene/propylene copolymer |
| 0.40 | Polymethacrylate |
| 0.10 | Phosphorus as neopentylpolyol adduct of inorganic phosphorus acid free, steam hydrolyzed polyisobutene-$P_2S_5$ reaction product |
| 0.25 | Calcium as an overbased calcium sulfonate |
| 0.15 | Zinc as a zinc dialkyldithiophosphate |
| 0.25 | Alkylated diphenylamine |
| 0.25 | Alkylated phenol |
| 0.35 | Friction modifier |
| 150 ppm | Silicone polymer |
| Balance | Mineral oil (~320 SUS at 100°F) |

The following tests were employed to demonstrate the efficiency of the lubricating oil compositions of this invention:

Oldsmobile Sequence IID Test

The Oldsmobile Sequence IID test is detailed in "Multicylinder Test Sequence for Evaluating Automotive Engine Oils", ASTM Special Technical Publication under 315-H. This procedure is used to evaluate motor oils with respect to low temperature rusting characteristics, and was designed to relate particularly to short trip service under typical winter conditions in the upper Midwestern United States. Oldsmobile Sequence IID test performance criteria required by the API SF Service Classification are set forth in Table 4.

Oldsmobile Sequence IIID Test

The Oldsmobile Sequence IIID test is detailed in "Multicylinder Test Sequence for Evaluating Automotive Engine Oils", ASTM Special Technical Publication under 315-H. This procedure is used to evaluate the ability of motor oils to protect against high temperature oxidation, deposits and wear. The test was designed to relate particularly to high speed motorway operation under relatively high ambient temperature conditions typical of the Southern and Southwestern part of the United States. Oldsmobile Sequence IIID test performance criteria required by the API SF Service Classification are set forth in Table 4.

Ford Sequence V-D Test

The Ford Sequence V-D test is detailed in "Multicylinder Test Sequence for Evaluating Automotive Engine Oils", ASTM Special Technical Publication under 315-H. This procedure is used to evaluate crankcase motor oils with respect to sludge and varnish deposits protection as well as their ability to protect against excessive valve train wear. This test was designed to simulate a combination of low speed, low temperature, "stop and go" city driving and moderate speed turnpike operation. The Ford Sequence V-D test criteria required by the API SF Service Classification are set forth in Table 4.

CRC L-38 Test

The Coordinating Research Council L-388 Test is detailed in the Federal Test Method Standard No. 791A, Method 3405.2. This test provides a method for evaluating the oxidation and copper-lead bearing corrosion characteristics of crankcase oils. The CRC L-38 test performance criterion required by the API SF Service Classification is set forth in Table 4.

Caterpillar 1H2 Test

The Caterpillar 1H2 test is detailed in "Single Cylinder Engine Tests for Evaluating the Performance of Crankcase Lubricants", ASTM Special Technical Publication 509A.

This procedure is used to evaluate the effectiveness of diesel engine crankcase lubricants in inhibiting the formation of piston deposits, preventing ring sticking and preventing wear. The Caterpillar 1H2 performance criteria required by the API CC Service Classification are set forth in Table 4.

Example 41

The lubricating compositions given in Examples 39 and 40 were evaluated in one or more of the abovementioned performance tests. The results are set forth in Table 4 below.

TABLE 4
The Evaluation of Lubricating Oil Compositions in Engine Tests

| Oils | Example 39 | Example 40 | API SF/CC Limits |
|---|---|---|---|
| *Sequence IID Test* | | | |
| Average engine rust rating | 8.8 | — | 8.5 min. |
| Lifter sticking | none | — | none |
| *Sequence IIID Test* | | | |
| Viscosity at 40°C increase at 64 hr, % | 28 | 55 | 375 max. |
| Average engine sludge | 9.6 | 9.6 | 9.2 min. |
| Average piston skirt varnish | 9.2 | 9.3 | 9.2 min. |
| Average oil ring land deposit | 8.0 | 7.0 | 4.8 min. |
| Cam plus lifter wear | | | |
| average, mm | 0.0533 | 0.0584 | 0.1016 max. |
| maximum, mm | 0.0889 | 0.0737 | 0.2032 max. |
| Cam or lifter scuffing | none | none | none |
| Ring sticking | none | none | none |
| Lifter sticking | none | none | none |
| Oil consumption, litres | 3.94 | 3.94 | 5.03 max. |
| *Sequence V—D Test* | | | |
| Average engine sludge | 9.7 | — | 9.4 min. |
| Average piston skirt varnish | 7.2 | — | 6.7 min. |
| Average engine varnish | 6.9 | — | 6.6 min. |
| Cam wear | | | |
| average, mm | 0.0152 | — | 0.0254 max. |
| maximum, mm | 0.0178 | — | 0.0635 max. |
| Oil ring clogging, % | 0 | — | 10.0 max. |
| Oil screen clogging, % | 0 | — | 7.5 max. |
| Compression ring sticking | none | — | none |
| *L—38 Bearing Test* | | | |
| Bearing weight loss, mg | 24.3 | — | 40 max. |
| *Caterpillar 1H2 Test* | | | |
| *120 hour* | | | |
| Top groove fill, % | 13.0 | — | 4.0 max. |
| Weighted total demerits | 25.6 | — | 71.9 max. |
| *480 hour* | | | |
| Top groove fill, % | 15.5 | — | 45 max. |
| Weighted total demerits | 96.5 | — | 140 max. |

The foregoing tests demonstrate that the prescribed lubricating oil formulations prepared from the synthetic lubricant component exhibit a high level of performance in both gasoline and diesel engines, and meet the API SF/CC limits in every respect.

The oligomer mixtures produced from $C_{13-14}$ internal olefins via a promoted $BF_3$ catalyst have proven to be exceptional synthetic lubricant additives. As these mixtures have a viscosity of about four centistokes at 99°C, they are considered "4 centistoke" fluids. A typical fluid of this invention was compared with the commercially available 4 centistoke decene-1 derived polyalpha olefin (PAO). It should be emphasized that the synthetic lubricant components of the present invention are preferably used without further distillation after the monomer removal and hydrogenation steps. In other words, the undistilled bottoms are the finished synthetic lubricant component. The polyalpha olefins must be distilled into "2 centistoke", "4 centistoke", "6 centistoke", etc. fractions before they can be useful. Thus, the method of this invention does not require a costly distillation step, which is an important advantage over methods used by others in the field. Comparisons of the properties of the fluids themselves are given in Table 5. It may be seen that the fluid of this invention (A) and the commercially available four centistoke decene-1 derived PAO (B) are generally comparable.

TABLE 5
Physical Properties of 4 Centistoke Fluids

| Test | A[1] | B[2] |
|---|---|---|
| Gravity, $g/cm^3$ | 0.821 | 0.820 |
| Flash, COC[2], °C | 220 | 225 |
| Kinematic viscosity, cSt 40°C | 17.0 | 17.1 |
| 100°C | 3.81 | 3.90 |
| Viscosity index | 115 | 123 |
| CCS viscosity, cP, −30°C | 980 | 930 |
| Brookfield viscosity, cP, −40°C | 2340 | 2200 |
| −28.9°C | 800 | 780 |
| ASTM color | 0.0 | 0.0 |
| Pour, °C[3] | <−55[4] | <−55[4] |
| Ash, % | 0.001 | 0.001 |

[1] Fluid of this invention.
[2] Commercially available 4 cSt decene-1 derived polyalpha olefin.
[3] Cleveland Open Cup.
[4] The actual pour points of the synthetic base oils were less than −55°C the lowest temperature at which pour points could be measured with the equipment available.

A measure of the volatility of the two fluids is presented in Table 6 where it may be seen that oligomer A is slightly more volatile than fluid B.

TABLE 6

| Test | A | B |
|---|---|---|
| ASTM Evaporation Test % loss, 205°C/6.5 hr | 16.9 | 14.8 |
| Thermal Gravimetric Analysis ß remaining at 233°C | 91 | 92 |
| Simulated Distillation % off column, °C | | |
| IBP | 364 | 326 |
| 2 | 377 | 392 |
| 4 | 381 | 405 |
| 6 | 384 | 408 |
| 8 | 386 | 411 |
| 10 | 388 | 412 |
| 15 | 391 | 413 |
| 20 | 394 | 414 |
| 25 | 396 | 418 |
| 50 | 405 | 427 |

A lubricating oil composition was formulated from the commercially available 4 centistoke decene-1 derived PAO for comparison with the lubricating oil composition of this invention. This formulation is shown in Example 42. Except for the 4 centistoke fluid, this oil contains the same proportions of the same supplemental base oil and additives that were employed in the lubricating oil composition of Example 39. That is, the rest of the components of the two formulations are identical in type and proportion and include materials such as mineral oils, dispersants, anti-oxidants, detergents, friction modifiers, rust inhibitors, viscosity index improvers, pour point depressants and anti-foamants. The proportion of 4 centistoke fluid (35 wt.%) is large enough to have a profound effect on the behaviour of the formulations in testing.

Example 42 (for comparison)

A SAE 5W—30 grade lubricating oil composition was derived by combining the following components:

| Weight Percent | |
|---|---|
| 35.00 | Four centistoke commercially available decene-1 derived synthetic lubricant component |
| 0.51 | Dispersant olefin copolymer VI improver |
| 0.08 | Polymethacrylate |
| 0.04 | Nitrogen as an alkenyl succinimide |
| 0.24 | Calcium as an overbased calcium sulfonate |
| 0.12 | Zinc as a zinc dialkyldithiophosphate |
| 0.25 | Alkylated diphenylamine |
| 0.25 | Alkylated phenol |
| 0.05 | Ethoxylated alkylphenol |
| 0.50 | Friction modifier |
| 150 ppm | Silicone polymer |
| Balance | Mineral oil (~300 SUS at 38°C) |

The results of the engine tests of the two formulations are presented in Table 7 below.

TABLE 7
Engine Test Results

| Tests | Example 39 | Example 42 | API SF/CC Limits |
|---|---|---|---|
| *Sequence IID Test* | | | |
| Average engine rust rating | 8.8 | 8.5 | 8.5 min. |
| Lifter sticking | none | none | none |
| *Sequence IIID Test* | | | |
| Viscosity at 40°C increase at 64 hr, % | 28 | 242 | 375 max. |
| Average engine sludge | 9.6 | 9.6 | 9.2 min. |
| Average piston skirt varnish | 9.2 | 9.2 | 9.2 min. |
| Average oil ring land deposit | 8.0 | 7.1 | 4.8 min. |
| Cam plus lifter wear | | | |
| average, mm | 0.0523 | 0.0279 | 0.1016 max. |
| maximum, mm | 0.0889 | 0.0686 | 0.2032 max. |
| Cam or lifter scuffing | none | none | none |
| Ring sticking | none | none | none |
| Lifter sticking | none | none | none |
| Oil consumption, litres | 3.94 | 5.10 | 5.03 max. |
| *Sequence V—D Test* | | | |
| Average engine sludge | 9.7 | 9.6 | 9.4 min. |
| Average piston skirt varnish | 7.2 | 6.8 | 6.7 min. |
| Average engine varnish | 6.9 | 6.6 | 6.6 min. |
| Cam wear | | | |
| average, mm | 0.0152 | 0.0076 | 0.0254 max. |
| maximum, mm | 0.0178 | 0.0102 | 0.0655 max. |
| Oil ring clogging, % | 0 | 0 | 7.5 max. |
| Oil screen clogging, % | 0 | 0 | 10.0 max. |
| Compression ring sticking | none | none | none |
| *L—38 Bearing Test* | | | |
| Bearing weight loss, mg | 24.3 | 45.9<br>39.5 | 40 max. |
| *Caterpillar 1H2 Test* | | | |
| Top groove fill, % | 5.5 | 5.0 | 45 max. |
| Weighted total demerits | 96.5 | 137.1 | 140 max. |

Sequence IIID tests are oxidation stability tests designed to measure the wear protection characteristics; that is, to measure how the oils protect internal loaded engine components against excessive wear. The engine is run at high speed under high loads. The first measurement is the percent increase in 40°C viscosity after 64 hours. It is surprising to note that the formulation of Example 39 had a much lower viscosity change than did that of Example 42, when it is remembered that the oligomer mixture of Example 42 had a higher viscosity index (indicating less change of viscosity with temperature) than did that of Example 39 (see Table 5). What is also surprising is that the formulation of Example 42 required 5.10 litres of oil for the Sequence IIID tests, whereas that of Example 39 required only 3.94 litres. This result is contrary to what would be expected from the volatility information derived from the simulated distillation set out in Table 6. It is particularly surprising when it is realized that the 4 cs fluid of this invention comprises only about 25% heavies. One skilled in the art would expect the more volatile material (A) to cause any formulation made therefrom to require more oil. However, as may be seen in Table 5, it is formulation B, having the less volatile oligomer mixture, which has the greater quantity requirements.

In the rest of the sequence IIID tests, the two formulations were quite comparable. However, the formulation A of Example 39 again gives better results in the Coordinating Research Council L-38 test, which is a measure of bearing corrosion by measuring the weight loss of the bearing. In the first determination, the formulation of Example 42 failed the test with a weight loss of 45.9 mg, though it passed on a second attempt with 39.5 mg loss. The formulation of Example 39 caused a much lower weight loss of 24.3 mg.

The Caterpillar 1H2 test measures the accumulation of deposits on diesel pistons, among other criteria. Demerits are assigned in this test by the subjective opinion of a person approved by API (American Pertroleum

Institute). Although the formulation of Example 39 performed poorer than that of Example 42 in the amount of top groove filling found, the weighted total demerits for both durations for Example 39 were much less than that for Example 42. These results are further evidence of the surprisingly superior characteristics of the oligomer mixtures made from only internal olefins and a promoted $BF_3$ catalyst.

## Claims

1. A process for the oligomerization of mono olefins by contacting a mixture of mono olefins with a catalyst, wherein said mono olefins comprise a mixture containing at least 99 weight% of internal mono olefins having 9 to 24 carbon atoms and having their double bond randomly distributed in their carbon chain, and the catalyst comprises boron trifluoride characterized by the use of a promoter for the catalyst which promoter is 1-butanol and further characterized in that the boron trifluoride is added to an autoclave containing the said olefins and 1-butanol in a weight ratio of boron trifluoride to 1-butanol of 1.57:1 to 4:1, and further characterized in that the mixture of mono olefins is a mixture of mono olefins having 13 or 14 carbon atoms.

2. A process according to Claim 1 characterized in that oligomerization is conducted at a temperature from 25 to 150°C.

3. A process according to any one of the preceding Claims characterized in that oligomerization product is subsequently hydrogenated.

4. A lubricant composition comprising a major amount of oligomerized olefins and minor amounts of lubricant additives, characterized in that the oligomerized olefins comprise an oligomerized product obtainable by a process according to any one of Claim 1 to 3 and in that the composition contains 10 to 40 weight% of the oligomerized olefins.

5. A lubricant composition according to Claim 4, characterized in that the oligomerized olefins have a viscosity at 99°C of 3.5 to 5.0 centistokes, a viscosity at 25°C of 25 to 40 centistokes, a viscosity index of at least 100 and a thermogravimetric analysis value of greater than 80 weight%.

6. A lubricant composition according to either Claim 4 or 5 characterized in that the additives comprise:-

i) from 0.1 to 15.0 wt% of viscosity index improver.

ii) from 0.01 to 10 wt% on an oil free basis of detergent/ dispersant system.

iii) from 0.01 to 3.0 wt% of oxidation and wear inhibitors.

iv) from 10 to 1,000 parts per million of anti-foamant, and

v) optionally one or more friction modifiers, rust inhibitors or pour point depressants

## Patentansprüche

1. Verfahren zur Oligomerisierung von Monoolefinen durch Kontaktieren einer Mischung aus Monoolefinen mit einem Katalysator, worin Jene Monoolefine aus einer Mischung mit mindestens 99 Gew.-% an Innenmonoolefinen mit 9 bis 24 Kohlenstoffatomen, deren Doppelbindung statistisch in der Kohlenstoffkette verteilt ist, bestehen, und der Katalysator aus Bortrifluorid besteht, gekennzeichnet durch Verwendung eines Promotors für den Katalysator, bei dem es sich um 1-Butanol handelt, und weiterhin dadurch gekennzeichnet, daß das Bortrifluorid in einen, Jene Olefine und 1-Butanol enthaltenden Autoklaven in einem Gewichtsverhältnis von Bortrifluorid zu 1-Butanol von 1,57:1 zu 4:1 gegeben wird, und weiterhin dadurch gekennzeichnet, daß es sich bei der Mischung aus Monoolefinen um eine Mischung aus Monoolefinen mit 13 oder 14 Kohlenstoffatomen handelt.

2. Verfahren nach Anspruch 1, dadurch gekennzeichnet, daß die Oligomerisierung bei einer Temperatur von 25 bis 150°C erfolgt.

3. Verfahren nach einem der vorhergehenden Ansprüche, dadurch gekennzeichnet, daß das Oligomerisierungsprodukt anschließend hydriert wird.

4. Schmiermittelzusammensetzung aus einer größeren Menge an oligomerisierten Olefinen und geringeren Mengen an Schmiermittelzusatzstoffen, dadurch gekennzeichnet, daß die oligomerisierten Olefine aus einem oligomerisierten Produkt bestehen, das nach einem, Verfahren nach einem der Ansprüche 1 bis 3 erhältlich ist und daß die Zusammensetzung 10 bis 40 Gew.-% oligomerisierter Olefine enthält.

5. Schmiermittelzusammensetzung nach Anspruch 4, dadurch gekennzeichnet, daß die oligomerisierten Olefine eine Viskosität bei 99°C von 3,5 bis 5,0 Zentistokes, eine Viskosität bei 25°C von 25 bis 40 Zentistokes, einen Viskositätsindex von mindestens 100 und einen thermogravimetrischen Analysenwert von mehr als 80 Gew.-% aufweisen.

6. Schmiermittelzusammensetzung nach Anspruch 4 oder 5, dadurch gekennzeichnet, daß die Zusatzstoffe:

i) 0,1 bis 15,0 Gew.-% eines Viskositätsindexverbesserers,

ii) 0,01 bis 10 Gew.-% bezüglich einer ölfreien Basis eines Detergens/Dispergiermittelsystems,

iii) 0,01 bis 3,0 Gew.-% oxidations- und abtragshemmender Mittel,

iv) 10 bis 1000 Teile pro Million eines Schaumverhinderungsmittels, und

v) gegebenenfalls ein oder mehrere reibungsverändernde Mittel, Rostschutzmittel oder Pourpoint-Depressoren enthalten.

## Revendications

1. Procédé d'oligomérisation de mono-oléfines en mettant en contact un mélange de mono-oléfines avec un catalyseur, dans lequel ces mono-oléfines comprennent un mélange contenant au moins 99 % en poids de mono-oléfines internes ayant 9 à 24 atomes de carbone et dont les doubles liaisons sont réparties au hasard dans leur chaîne carbonée, et le catalyseur comprend du trifluorure de bore, caractérisé en ce que l'on utilise un promoteur pour le catalyseur, lequel promoteur est du 1-butanol, et caractérisé en outre en ce que le trifluorure de bore est ajouté dans un autoclave contenant ces oléfines et le 1-butanol dans un rapport pondéral du trifluorure de bore au 1-butanol de 1,57:1 à 4:1, et caractérisé en outre en ce que le mélange de mono-oléfines est un mélange de mono-oléfines ayant 13 ou 14 atomes de carbone.

2. Procédé selon la revendication 1, caractérisé en ce que l'oligomérisation est effectuée à une température de 25 à 150°C.

3. Procédé selon l'une quelconque des revendications précédentes, caractérisé en ce que le produit de l'oligomérisation est ensuite hydrogéné.

4. Composition de lubrifiant comprenant une quantité prédominante d'oléfines oligomérisées et des quantités mineures d'additifs de lubrifiant, caractérisée en ce que les oléfines oligomérisées comprennent un produit oligomérisé pouvant être obtenu par un procédé selon l'une quelconque des revendications 1 à 3 et en ce que la composition contient 10 à 40 % en poids des oléfines oligomérisées.

5. Composition de lubrifiant selon la revendication 4, caractérisée en ce que les oléfines oligomérisées ont une viscosité à 99°C de 3,5 à 5,0 centistockes, une viscosité à 25°C de 25 à 40 centistockes, un indice de viscosité d'au moins 100 et une valeur d'analyse thermogravimétrique supérieure à 80 % en poids.

6. Composition de lubrifiant selon l'une quelconque des revendications 4 ou 5, caractérisée en ce que les additifs comprennent :

1) de 0,1 à 15,0 % en poids d'un agent d'amélioration de l'indice de viscosité,

2) de 0,01 à 10 % en poids d'un système détergent/dispersant sur la base du produit exempt d'huile,

3) de 0,01 à 3,0 % en poids d'inhibiteurs d'oxydation et d'usure,

4) de 10 à 1 000 parties par million d'un anti-mousse et,

5) si on le désire un ou plusieurs modificateurs de frottement, antirouille ou abaisseurs du point d'écoulement.